# EUROPEAN PATENT SPECIFICATION

(11) **EP 1 553 885 B1**
(45) Date of publication and mention of the grant of the patent: **30.05.2007**
(21) Application number: 03748430.0
(22) Date of filing: 09.10.2003
(51) Int. Cl.: A61B 18/18

(54) **INTERSTITIAL MICROWAVE ANTENNA WITH LATERAL EFFECT FOR TISSUES HYPERTHERMIA IN MINIMAL INVASIVE SURGERY**
INTERSTITIELLE SEITLICH WIRKENDE MIKROWELLENANTENNE FÜR GEWEBEHYPERTHERMIE IN MINIMALINVASIVER CHIRURGIE
ANTENNE HYPERFREQUENCE INTERSTITIELLE AVEC EFFET LATERAL POUR HYPERTHERMIES TISSULAIRES EN CHIRURGIE INVASIVE MINIMALE

(30) Priority: 22.10.2002 IT PI20020059
(43) Date of publication of application: 20.07.2005
(73) Proprietor: Longo, Iginio, 56123 Pisa (IT)
(72) Inventor: LONGO, Iginio, I-56123 Pisa (IT); TOSORATTI, Nevio, I-00151 Roma (IT)
(74) Representative: Celestino, Marco
(86) International application number: PCT/IB2003/004459
(87) International publication number: WO 2004/037102

(56) References cited:
- WO-A-95/10253
- US-A- 5 456 662
- US-A- 5 599 294
- US-A- 6 044 846
- US-A1- 2002 058 932

## Description

### Field of the invention

The present invention relates to mininvasive surgery techniques, for hyperthermia of solid deep wounds for interstitial, percutaneous, laparoscopic, endoscopic and intra-operation applications in medicine and surgery, especially in oncology. More precisely, the present invention relates to a microwave co-axial antenna particularly indicated for hyperthermia of large tissue masses. Furthermore, the invention relates to a method for manufacturing such an antenna.

### Description of the prior art

Hyperthermia in oncology is a method that has been used for over 30 years for treatment of cancer (Hahn GM, Hyperthermia and Cancer, Plenum Press, in the York, 1982). It consists in heating cancer cells to obtain their necrosis either directly or with additional use of other methods such as radiotherapy, chemotherapy or other surgery techniques.

For heating tissues, in particular for treatment of surface lesions, firstly electromagnetic waves have been used produced by a source located out of the human body.

More recently thin appliances have been used among which microwave antennas, operating between several hundreds of MHz and several thousands of MHz, typically at 2450 MHz, created with a co-axial tube, for interstitial, percutaneous, laparoscopic, endoscopic and intra-operation applications, suitable for the local treatment of deep lesions (Iskander MF & Tumeh AM, Design Optimization of Interstitial Antennas, IEEE Transactions on Biomedical Engineering, 1989, 238-246).

Such antennas are usually inserted within the lesion to treat using catheters or metal needles, under computerised imaging techniques such as echographic guiding, TAC, NMR or other. They are suitable for being used in combination with other actions such as drugs, ionizing waves and/or surgery ablation.

These microwave antennas, normally, are manufactured using a flexible or semi-rigid co-axial tube, suitably modified at one end, for conveying microwave power into the tissues to cause hyperthermia.

The use of the minimally invasive microwaves coagulation therapy (TCMM) for percutaneous, laparoscopic applications, etc., is well known and widely documented in many industrial extra-European countries (USA, Japan, Canada, China, etc.).

Such therapy provides introducing a small diameter co-axial antenna up to directly the centre of a lesion, of cancerous or hypertrophic tissue, normally through a introduction metal needle or a plastic catheter.

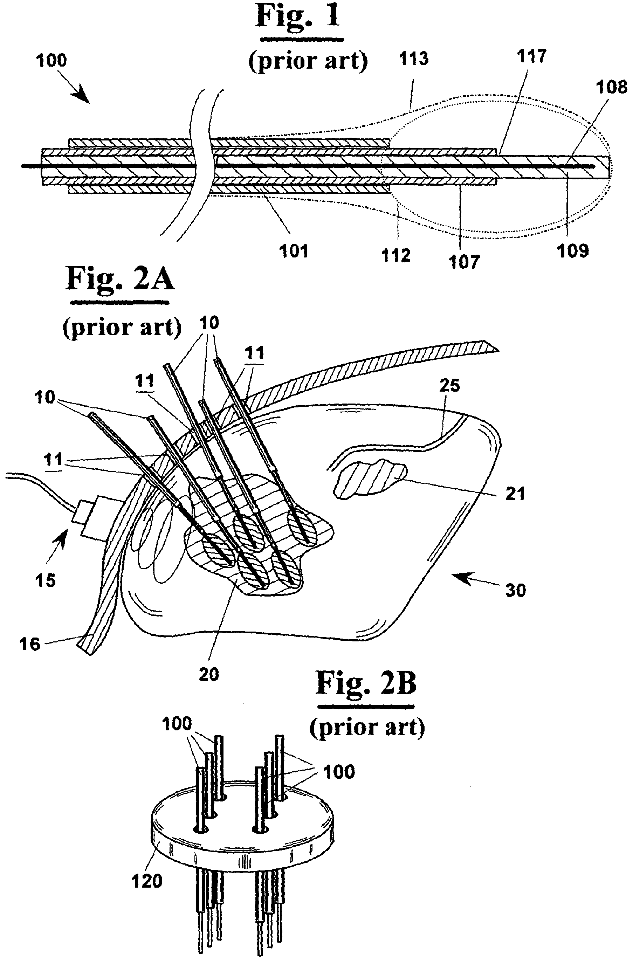

In figure 1 an axial cross section is shown of an antenna 100 integrated with a biopsy needle 100 of known art. The active part of the antenna, in the right side of the drawing, is suitably configured as a radiating dipole or monopole. More precisely, 107 is the external conductor of the co-axial tube, 109 is the dielectric layer that insulates the external conductor from the central conductor 108. Isothermal surfaces having a rotationally symmetric configuration can be obtained by heating a biological tissue (not crossed by large blood vessels) with a normal antenna 100, that for example is made by cutting at an end the portion of the external conductor 107 of the co-axial tube and leaving dielectric layer 109 uncovered as described in figure 1.

Once put within the lesion, the active end of the antenna exceeding the needle 101 emits microwave power (typically 60 W, at the frequency of 2450 MHz) sufficient to obtain in a few minutes the necrosis of a mass of tissue of spheroidal shape 112: for example, for coagulating 10 ml of aqueous tissue 2-3 minutes are required. The coagulative necrosis induced by the treatment destroys the tissue, which, normally, remains in its position where it is subject to a fibrotic process, it shrinks and does not affect further the adjacent zones. However, as the duration of the treatment and/or of the power supplied by the microwave antenna increase, the coagulated mass does not grow proportionally its volume, since the subtraction of heat by the blood circulation and by diffusion for conductivity increases proportionally to the surface of the treated volume: the consequence is that with an antenna of conventional type it is possible to treat, in a single application, lesions with a diameter not more than 2-3 cm.

With the existing technology, to treat lesions of larger diameter (>3 cm), the application has to be repeated through successive insertions of a single antenna 100 as shown in figure 2A, or the simultaneous introductions of several antennas 100. As shown in figure 2B, in this case the use is known of a multiple support 120 to guide together all the needles (as an array). In both cases, the traumatic aspects of the hyperthermia treatments and the soreness immediately felt by the patient increase substantially.

It must be noted that if a single TCMM application is sufficient for treatment of a lesion of 3 cm of diameter, a lesion of 8 cm of diameter requires between 20 and 30 single operations, considering a 1 cm overlap safety factor. Then, the use of an array of antennas is justified only if the lesion can be treated with not too many antennas, since otherwise the rate of invasivity is, in fact, comparable to that of a conventional surgical operation, and the same happens in case of a treatment with a large number of consecutive insertions of a single applicator in different points.

Always as shown in figure 2A, by treating hepatic lesions in a percutaneous way using antenna 100 of conventional type, whereas lesion 20 can be treated even if requiring numerous insertions for coagulating the whole mass, it is not instead possible to treat lesion 21 next to a large blood vessel 25 for high risk of perforating or coagulating the vessel same.

Furthermore, lesions of irregular shape or that cannot be crossed longitudinally by the applicator are a further difficulty for conventional applicators presently in use. In patent number US 5599294 a medical probe device is disclosed comprising a catheter having a stylet guide housing with one or more stylet ports in a side wall thereof and guide means for directing a flexible stylet outward through the stylet port and through the tissue at a preselected, adjustable angle to a target tissue. The total catheter assembly includes a stylet guide lumen communicating with the stylet port and a stylet positioned in said stylet guide lumen for longitudinal movement from the port through intervening tissue to a target tissue.

### Summary of the invention

It is a feature of the present invention to provide a microwave co-axial antenna for applications in medicine and surgery for further reducing treatment invasivity of a minimally invasive microwaves coagulation therapy with respect to the prior art, avoiding both a multiplication of the number of applications required for treating large lesions with a single antenna of conventional design (through repeated extraction and reintroduction of the applicator at different points of the tissue) both a requirement of an array of antennas.

It is also a feature of the present invention to provide an antenna that is inserted into a lesion and proceeds in a direction lateral/oblique with respect to the axis of the application needle and with an adjustable angle.

It is a further disclosed a method for the production of such an antenna and of its application device.

These and other features are accomplished with one exemplary antenna according to claim 1. Preferred embodiments are described in the dependent claims.

Preferably, the needle is a metal needle which, in the end portion, has a stiff blocking material, for example metal, having a tapered inner face forming said chute guide and a sharp external face.

Alternatively, the needle in the end portion has a gradually increasing thickness in order to form said chute guide.

Advantageously, for allowing the introduction of the antenna in the target tissue along the actuation direction a metal flexible mandrel is provided sliding in the needle before introducing the antenna and suitable for protruding from it through the side opening for making an inlet hole in the tissue to treat according to the actuation direction.

Once made the hole along the actuation direction, the mandrel is extracted from the needle and replaced by the co-axial antenna that is inserted in the hole made previously by the mandrel for a length suitable for allowing a correct operation. When the treated zone has reached a predetermined temperature, the antenna is withdrawn and can be inserted then in another position after having rotated the needle a certain angle or after having translated it along the introduction direction, without the need of making other inlet holes. This way, it is possible to apply hyperthermia to a mass of tissue having an axial symmetry or to masses of irregular shape.

Furthermore, such an antenna allows to heat lesions located laterally with respect to the needle without that these are necessarily crossed by the needle. This feature allows to treat lesions extending near large blood vessels, that cannot be treated with the antennas of prior art owing to a high risk of perforating the vessel.

In particular, it is possible to replace the needle with another having a different angle α for the actuation direction with respect to the introduction direction. Within certain limits, the length can also be changed of the portion of antenna exceeding the opening. This way, the shape of the area of operation can be changed as preferred.

### Brief description of the drawings

Further characteristics and advantages of the interstitial antenna, according to the present invention will be made clearer with the following description of an exemplary embodiment thereof, exemplifying but not limitative, with reference to the further attached drawings wherein:
- figures 3 and 4 show a cross sectional axial view of an application device for an interstitial antenna, according to the invention;
- figures 5 and 6 show in axial cross section an interstitial antenna, according to the invention;
- figure 7 shows a cross sectional axial view of an alternative exemplary embodiment for the application device of figure 3;
- figure 8 shows diagrammatically a possible use of the interstitial antenna of figures 5 and 6.

### Description of a preferred exemplary embodiment

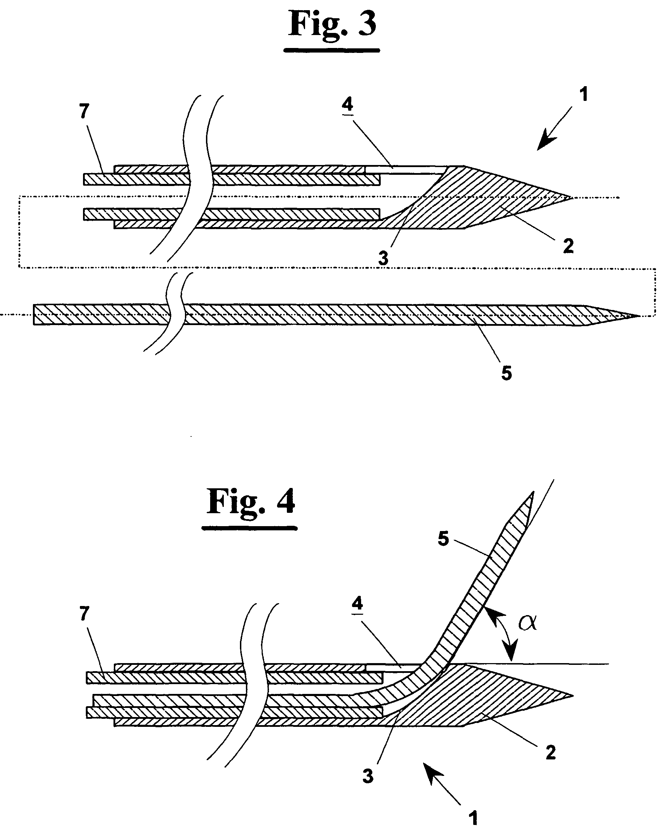

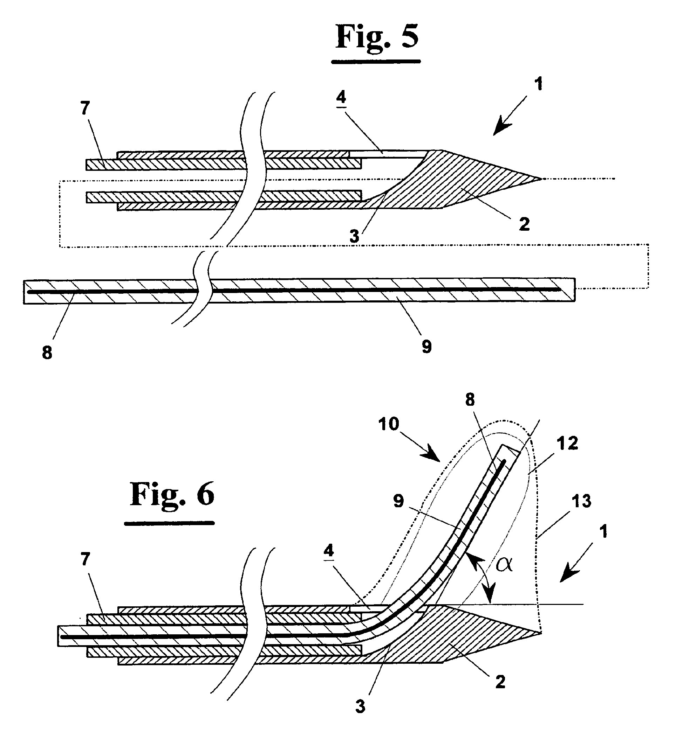

In figure 3, an application device 1 according to the invention is shown, in an axial cross section, for example a metal needle that has in the end portion 2 of its free end a gradually increasing thickness to form substantially a chute guide 3 which ends at a side opening 4 made on application device 1. This way, an interstitial antenna 10 is obtained (figure 6) formed by a co-axial tube having an external conductor 7, by a dielectric cable 9 and by a central conductor 8 embedded in the dielectric cable 9 that insulates it from the external conductor 7. The antenna 10 can be put in a target tissue, along an actuation direction forming an angle α with the introduction direction.

With reference to figure 4, for allowing the introduction of cable 9 in the target tissue along the actuation direction a metal flexible mandrel is provided 5, suitably shaped, sliding in application device 1 and capable of protruding from it through the side opening 4 for making an inlet hole in the tissue to treat according to the actuation direction forming an angle α with the introduction direction.

Then, once made the hole along the actuation direction, the mandrel 5 is extracted from application device 1 and replaced by cable 9 that is inserted in the hole made previously by the mandrel 5 for a length suitable for allowing a correct operation (figure 6).

In particular, a calculated isothermal surface obtained by the antenna is indicated in figure 6 by a curved dashed line 12, whereas the actual isothermal surface, i.e. the mass of tissue actually coagulated is enclosed within a curved line 13, since the tip of application device 1 is connected electrically with the external conductor 7 and increases its area of action.

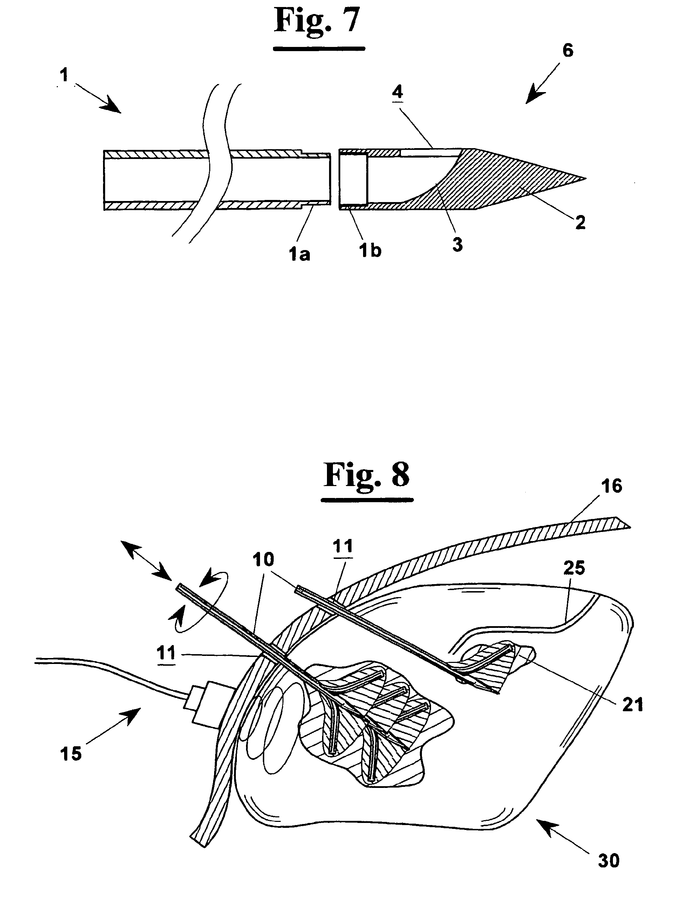

When the treated zone has reached a predetermined temperature, cable 9 is withdrawn and it can be put forward again after having rotated application device 1 of a certain angle or after having translated it along the introduction direction, without the need of making other inlet holes 11, as shown in figure 8. It is sufficient to repeat the above operations with mandrel 5 and then cable 9. This way, it is possible to reduce remarkably the treatment invasivity avoiding both to multiply the number of applications required for treating large lesions 20 with a single antenna 100 of conventional design (figure 2A) both to use an array of antennas (figure 2B), which requires a single hole but of much larger diameter.

Furthermore, the antenna 10, according to the invention, allows to heat lesions located laterally with respect to application device 1 without that they are crossed by a hole. This feature allows to treat lesions 21 extending near a large blood vessel 25, that cannot be treated with the antennas of prior art (figure 2A), for high risk of perforating the vessel 25 same.

In figure 7 an alternative exemplary embodiment is shown for application device 1. This embodiment provides that the metal needle 1 are associated to a block 6 of stiff material, for example metal, having a side opening 4, a tapered inner face forming the chute guide 3 and a sharp external face. Needle 1 and block 6 are releasably engaged, for example, by a screw threaded coupling 1a-1b. This way, it is possible to change as desired the shape of the area of operation simply replacing the block 6 for adjusting the angle α of the actuation direction with respect to the introduction direction.

The foregoing description of a specific embodiment will so fully reveal the invention according to the conceptual point of view, so that others, by applying current knowledge, will be able to modify and/or adapt for various applications such an embodiment without further research and without parting from the invention, and it is therefore to be understood that such adaptations and modifications will have to be considered as equivalent to the specific embodiment. The means and the materials to realise the different functions described herein could have a different nature without, for this reason, departing from the field of the invention. It is to be understood that the phraseology or terminology employed herein is for the purpose of description and not of limitation.

## Claims

1. Microwave device (1) for interstitial, percutaneous, laparoscopic, endoscopic and intra-operation applications in medicine and surgery, in species for applications of acute hyperthermia in oncology, comprising :
- an inner conductor (8),
- a dielectric layer (9) that covers said inner conductor (8) for all its length,
- an external conductor (7) that covers coaxially said dielectric layer (9) except from an end portion, forming together with said dielectric layer (9) and said inner conductor (8) a co-axial antenna (10),
- a hollow needle (for coaxially guiding said antenna (10) in a target tissue along an introduction direction,
**characterised in that** said needle has in the end portion (2) a side opening (4) and a chute guide (3), said chute guide (3) guiding said antenna (10) through said side opening (4) causing it to enter the target tissue along an actuation direction that forms an angle α with respect to said needle.

2. Microwave device for interstitial applications, according to claim 1, wherein said needle is a metal needle having in the end portion (2) a block of stiff material, for example metal, having a tapered inner face forming said chute guide (3) and a sharp external face.

3. Microwave device for interstitial applications, according to claim 1, wherein said needle is blind in the end portion (2) and at said side opening (4) it provides a gradually increasing thickness in order to form said chute guide (3).

4. Microwave device for interstitial applications, according to claim 1, wherein for the introduction of the antenna (10) in the target tissue along the actuation direction a metal flexible mandrel (5) is provided sliding in said needle before introducing the antenna (10) and suitable for protruding from it through said side opening (4) for making an inlet hole (11) in the tissue to treat according to said actuation direction.

## Patentansprüche

1. Mikrowellengerät (1) für interstitielle, perkutane, laparoskopische, endoskopische und intra-operative Anwendungen in der Medizin und Chirurgie an Spezies für die Anwendungen akuter Hyperthermie in der Onkologie, umfassend:
- einen inneren Leiter (8),
- eine dielektrische Schicht (9), welche den inneren Leiter (8) über seine gesamte Länge bedeckt,
- einen externen Leiter (7), der koaxial die dielektrische Schicht (9) außer an einem Endabschnitt bedeckt und zusammen mit der dielektrischen Schicht (9) und dem inneren Leiter (8) eine koaxiale Antenne (10) bildet,
- eine Hohlnadel für das koaxiale Führen der Antenne (10) in ein Zielgewebe entlang einer Einführrichtung,
**dadurch gekennzeichnet, dass** die Nadel in dem Endabschnitt (2) eine seitliche Öffnung (4) und eine Gleitführung (3) aufweist, wobei die Gleitführung (3) die Antenne (10) durch die seitliche Öffnung (4) führt und sie in das Zielgewebe entlang einer Betätigungsrichtung, die einen Winkel α in Bezug auf die Nadel bildet, eintreten lässt.

2. Mikrowellengerät für interstitielle Anwendungen nach Anspruch 1, wobei die Nadel eine Metallnadel ist, die in dem Endabschnitt (2) einen Block aus steifem Material, beispielsweise Metall, mit einer konisch zulaufenden Innenfläche, welche die Gleitführung (3) bildet, und einer spitz zulaufenden Außenfläche aufweist.

3. Mikrowellengerät für interstitielle Anwendungen nach Anspruch 1, wobei die Nadel im Endabschnitt (2) blind bzw, verschlossen ist und an der seitlichen Öffnung (4) eine allmählich zunehmende Dicke aufweist, um die Gleitführung (3) zu bilden.

4. Mikrowellengerät für interstitielle Anwendungen nach Anspruch 1, wobei für das Einführen der Antenne (10) in das Zielgewebe entlang der Betätigungsrichtung vor dem Einführen der Antenne (10) ein flexibler Metalldorn (5) verschiebbar in der Nadel vorgesehen ist und geeignet ist, von dieser durch die seitliche Öffnung (4) einzudringen, um ein Einlassloch (11) in das zu behandelnde Gewebe entlang der Betätigungsrichtung einzubringen.

## Revendications

1. Dispositif à hyperfréquence (1) pour des applications interstitielles, percutanées, laparoscopiques, endoscopiques et intra-opératoires en médecine et en chirurgie, dans les cas d'applications d'hyperthermie aiguë en oncologie, comprenant:
- un conducteur intérieur (8),
- une couche diélectrique (9) qui recouvre ledit conducteur intérieur (8) sur toute sa longueur,
- un conducteur externe (7) qui recouvre co-axialement ladite couche diélectrique (9) à l'exception d'une portion terminale, formant ensemble avec ladite couche diélectrique (9) et ledit conducteur intérieur (8) une antenne co-axiale (10),
- une aiguille creuse visant à guider co-axialement ladite antenne (10) dans un tissu cible selon un sens d'introduction,
**caractérisé en ce que** ladite aiguille comporte, dans la partie terminale (2) une ouverture latérale (4) et un guide d'éjection (3), ledit guide d'éjection (3) conduisant ladite antenne (10) par ladite ouverture latérale (4), la forçant à pénétrer dans le tissu cible selon un sens d'actionnement qui forme un angle α par rapport à ladite aiguille.

2. Dispositif à hyperfréquence pour des applications interstitielles selon la revendication 1, dans lequel ladite aiguille est une aiguille métallique comportant dans la partie terminale (2) un bloc de matériau rigide, par exemple un métal, ayant une face intérieure tronconique formant ledit guide d'éjection (3) et une face externe acérée.

3. Dispositif à hyperfréquence pour des applications interstitielles selon la revendication 1, dans lequel ladite aiguille est borgne dans la partie terminale (2) et au niveau de ladite ouverture latérale (4) il se crée une épaisseur qui augmente progressivement afin de former ledit guide d'éjection (3).

4. Dispositif à hyperfréquence pour des applications interstitielles selon la revendication 1, dans lequel, pour introduire l'antenne (10) dans le tissu cible selon le sens d'actionnement, on installe un mandrin flexible métallique (5) qui coulisse dans ladite aiguille avant d'introduire l'antenne (10) et qui est approprié pour former une protrusion à partir de l'aiguille par ladite ouverture latérale (4) de façon à former un orifice d'admission (11) dans le tissu pour traiter selon ledit sens d'actionnement.
